# EUROPEAN PATENT APPLICATION

(11) **EP 4 124 855 A1**
(43) Date of publication of application: **01.02.2023**
(21) Application number: 21382711.6
(22) Date of filing: 29.07.2021
(51) Int. Cl.: G01N 27/414, C12Q 1/70, G01N 33/48, C12N 15/11

(54) **GRAPHENE BIOSENSOR FOR THE DETECTION OF HEPATITIS C VIRUS**

(71) Applicant: Consejo Superior De Investigaciones Científicas (CSIC), 28006 Madrid (ES); INTA, Instituto Nacional de Técnica Aeroespacial, 28850 Torrejon de Ardoz (ES); INL - International Iberian Nanotechnology Laboratory, 4715-330 Braga (PT); Universidade do Minho, 4704-553 Braga (PT)
(72) Inventor: Vázquez Burgos, Luis Fernando, 28049 Madrid (ES); Palacio Rodríguez, Irene, 28049 Madrid (ES); Martín Gago, José Ángel, 28049 Madrid (ES); López Fagúndez, María Francisca, 28049 Madrid (ES); García Hernández, Mª del Mar, 28049 Madrid (ES); Briones Llorente, Carlos, 28850 Torrejón de Ardoz (Madrid) (ES); Moreno Molina, Miguel, 28850 Torrejón de Ardoz (Madrid) (ES); Nañez Cabrero, Almudena, 28850 Torrejón de Ardoz (Madrid) (ES); Torres Vázquez, Beatriz, 28850 Torrejón de Ardoz (Madrid) (ES); Alpuim, Pedro, 4715-330 Braga (PT); Domingues, Telma, 4715-330 Braga (PT); Cabral, Patrícia, 4715-330 Braga (PT)
(74) Representative: Pons

(57) **Abstract**

The present invention relates to an electrolyte-gated graphene field-effect transistor (G-FET) based biosensor characterized in that it comprises an aptamer against core protein of hepatitis C virus (HCV), particularly the aptamer comprising the nucleotide sequence SEQ ID NO:1, which allows the detection of core protein of HCV in the attomolar range both in buffer and in human plasma, greatly improving the sensitivity and specificity of the detection of HCV in biological samples.

## Description

The invention belongs to the field of instruments for measuring properties in electrolyte solutions, particularly belongs to the field of biodetection. Therefore, the invention relates to a biosensor for the detection of hepatitis C virus and uses therefor.

### BACKGROUND ART

Hepatitis C virus (HCV) affects 115 million people on five continents (in other words, 2-3% of the worldwide population) and 350,000 infected individuals die every year due to complications derived from hepatitis C (Pietschmann and Brown, 2019 Trends Microbiol., 27, 379-380). Currently, the diagnosis of the infection is performed by means of systems based on the detection of either the anti-HCV antibodies produced by the host (immunologic tests), or molecular components of the virus such as proteins or viral genomic RNA (antigenic tests). In the first case, there are different commercial ELISA (Enzyme-Linked ImmunoSorbent Assay) kits which can be used as a method of sieving. Nevertheless, they can give false negatives and that is why they require confirmation by means of another complementary technique, which is generally based on the detection of the viral genomic RNA using variants of the RT-PCR to specifically amplify the viral genome.

Biosensors have been developed for use in several fields of medical care, drug discovery, and clinical essays. Biosensors utilize the superior molecular identification ability of living organisms (or their parts) to recognize information from the outside world (such as chemical factors) as a physical signal, and various principles and measurement targets exist (Bhalla et al., 2016 Essays Biochem., 60, 1-8). In general, biosensors use biomolecules or artificial compounds that capture and identify other biomolecules (such as enzymes, structural proteins, antibodies or DNA), microorganisms or eukaryotic cells. Among the molecular probes used in biosensors, aptamers offer a number of advantages over antibodies. Aptamers are short, single stranded nucleic acid molecules (either ssDNA or RNA) which are obtained from combinatorial libraries by means of *in vitro* evolution protocols and acquire the ability to specifically bind any desired target molecule (Zhuo et al., 2017 Int. J. Mol. Sci., 18, 2142; Bayat et al., 2018 Biochimie, 154, 132-155). Therefore, a number of aptamer-based biosensors (also called aptasensors) have been developed. Among the different signal transduction systems used in biosensors, field-effect transistors (FETs) have been recently incorporated. FET-based biosensors are based on the conversion of a change in the chemical or physical state of the identifying biomolecule when bound to a target, into an electrical signal that can be measured.

FETs are characterized by containing three terminals (namely, the source, drain and gate electrodes) and the active channel which is formed by a semiconductive material. Through the active channel, e.g., formed by a semiconductive material, charge carriers (electrons or holes) flow from the source to the drain. The gate modulates the channel conductivity by applying a gate voltage to control the current between source and drain.

Nanoscale FETs which use single layers of graphite (graphene) as the semiconductive material in the active channel (Graphene field-effect transistors, G-FETs) and are analyte gated (whereby the system is closed by the addition of an analyte solution) have been developed for several applications in biotechnology and biomedicine. For example, the document WO2017216641 discloses Polar fluid gated FET devices used to detect chemicals/ions in fluids of the human body. Furthermore, G-FETs have been developed, among many other examples, for the detection of bacteria such as *Bacillus cereus* (Mohanty et al., 2008 Nano Letters, 8, 4469-4476) and *Escherichia coli* (Wu et al., 2017 Adv. Healthcare Mater., 1700736, 1-9), the detection of pH and protein adsorption (Ohno et al., 2009 Nano Letters, 9, 3318-3322) and Interferon-gamma (Farid et al., 2015 Biosensors and Bioelectronics, 71, 294-299).

Despite the recent advances in biosensors, no specific G-FET biosensor has been developed targeting specifically HCV.

### DESCRIPTION OF THE INVENTION

Given the above mentioned, the present invention uses G-FET-based aptasensors to solve the problems associated with the detection of HCV for clinical purposes. The present invention relates to an electrolyte-gated graphene field-effect transistor (G-FET) biosensor characterized in that it comprises an aptamer against HCV core protein, particularly the aptamer comprising the nucleotide sequence SEQ ID NO:1, which allows the detection of HCV core proteins in the attomolar range, greatly improving the sensitivity and specificity of HCV detection in biological samples.

The inventors have developed specific aptamers against molecular targets of this virus, specifically against the core protein, (which, by binding 24 monomers, forms the viral capsid) and investigated their applications both in HCV diagnosis and in therapy.

Therefore, a first aspect of the present invention is an electrolyte-gated graphene field-effect transistor (G-FET)-based biosensor, from here onwards the "biosensor of the invention", characterized in that said biosensor is functionalized with at least one aptamer comprising a nucleotide sequence according to SEQ ID NO:1.

In a preferred embodiment of the invention, the G-FET biosensor comprises:
a) an electrically insulating substrate (1);
b) a gate electrode (5) on the top of the electrically insulating substrate (1), wherein said gate electrode (5) contains at least one electrolyte-gated region (6) wherein an electrolyte (7) is arranged thereof;
c) a source electrode (2) and a drain electrode (3) on top of the electrically insulating substrate (1) and in the electrolyte-gated region (6), without direct contact between them (2, 3);
d) at least one graphene sheet (4) located between the source electrode (2) and the drain electrode (3) in the electrolyte-gated region (6) wherein said graphene sheet (4) is configured to electrically connect the source electrode (2) to the drain electrode (3);
wherein the electrolyte (7) is exposed to the graphene sheet (4) and the gate electrode (5);
characterized in that the graphene sheet is functionalized with at least one aptamer comprising a nucleotide sequence according to SEQ ID NO:1.

The G-FET is the basis for the biosensor of the invention. The term "biosensor" as used herein refers to an analytical device used to detect, as an example and without limitation, chemical substances, molecules, molecular aggregates, biomolecules, viral capsids, complete viruses, bacteria or eukaryotic cells, that combines a biological or biomimetic component (used as a probe molecule) with a physicochemical detector (signal transducer). In the present invention, the biological component is an aptamer and the physicochemical detector is the electrolyte-gated graphene field effect transistor (G-FET).

The expression "electrolyte-gated graphene field effect transistor" or its acronym "EGFET" or "G-FET" as used herein refer to a graphene channel between two conductive source-drain electrodes, which are typically metals, which are connected by one or more graphene sheets. Some portion of the graphene channel is exposed to the electrolytic environment, either directly or via some selectively permeable membrane. This allows changes in the electrolytic environment to alter the graphene sheet's electrical properties, which in turn alter the transistor channel conductance. A gate electrode is then used to apply a potential to the electrolytic environment which is electrically connected to the graphene sheets, enabling the identification of these changes in the transistor channel electrical properties. In the case of the present invention, the electrical properties of the graphene sheets can also be altered by binding the HCV core proteins to the aptamer covalently linked to the graphene sheets.

The base of the G-FET is the "substrate," which as used herein refers to any suitable material, such as: a semiconductor such as silicon, gallium arsenide; insulator nitrides such as boron nitride, aluminum nitride, aluminum oxynitride, silicon nitride, silicon oxynitride; insulator oxides such as silicon oxide, quartz, fused silica, glass, and spinon-glass, alumina, sapphire, hafnium oxide, hydrogen silsesquioxane; fiber materials such as carbon fiber and fiberglass; flexible plastic foils and coatings such as polyethylene naphthalate, polyethylene terephthalate, polyimide, polystyrene, polytetrafluoroethyle, polycarbonate, polymethylmethacrylate; resins such as epoxy resin, phenol-formaldehyde resin; rubbers such as polydimethylsiloxane, and composite and laminates of the above, such as epoxy-reinforced fiberglass, semiconductors coated or covered in an insulator or oxide. The term "substrate" may be used to define generally the elements for layers that underlie and/or overlie a layer or portions of interest.

In other preferred embodiment, the substrate of the biosensor of the invention is selected from the list consisting of: silicon, silicon oxide, silicon oxynitride and aluminium oxide.

The substrate may be formed by a conductive holder such as doped silicon, covered in a non-conductive material taken from the list above. Also, the substrate may be any other base on which a layer is formed, for example, a semiconductor wafer such as a silicon wafer or silicon chip. Several different substrates may be separate bodies with or without a mechanical connection between different substrates.

In the present invention, the substrate is an "electrically insulating substrate" referring to the fact that the substrate does not allow the flow of carriers, such as electrons or holes.

In a preferred embodiment of the biosensor of the invention, the electrically insulating substrate is silicon coated with silicon oxide or silicon nitride.

As stated, the electrolytic environment connects the graphene sheet and the gate electrode. The electrolytic environment is composed of an electrolyte solution. The term "electrolyte", as used herein, refers to a substance that produces an electrically conducting solution when dissolved in a polar solvent, such as water. The dissolved electrolyte is composed of cations and anions, which disperse uniformly through the solvent. Electrically, such a solution is neutral. If an electric potential is applied to such a solution, the cations of the solution are drawn to the electrode that has an abundance of electrons, while the anions are drawn to the electrode that has a deficit of electrons. The movement of anions and cations in opposite directions within the solution amounts to an electric current. Examples of an electrolyte solution are, without limitation, soluble salts, acids and bases, dissolved gases such as hydrogen chloride (HCI), biological fluids such as blood, plasma, urine, serum, saliva, mucus, interstitial fluid, amniotic fluid, lymphatic fluid, pericardial fluid, peritoneal fluid, and tears.

Therefore, in other preferred embodiment the electrolyte solution is selected from a list consisting of: blood, plasma, urine, serum, saliva, mucus, interstitial fluid, amniotic fluid, lymphatic fluid, pericardial fluid, peritoneal fluid, or tears.

The electrolyte is enclosed in the electrolyte-gated region in the biosensor of the invention. The expression "electrolyte-gated region" as used herein refers to a plannar region wherein the electrolyte solution can be in contact with both the graphene sheet and the gate electrode, i. e., the region where the electrolyte solution is placed. This region encloses at least the area of the exposed graphene sheets. Alternatively, if the gate electrode is composed of a planar surface, the electrolyte-gated region must also include the gate electrode. Particularly, the electrolyte-gated region can be a well, wherein said well encloses the electrolyte.

The biosensor of the invention comprises a gate electrode, a source electrode and a drain electrode. The term "electrode" as used herein refers to an electrical conductor used to make contact with a non-metallic part of a circuit. Electrodes can be made of several possible materials, such as, and without limitation, silver, gold, copper, carbon, graphite ink, conductive fabrics, conductive textiles, metals, conductive materials, conductive polymers, conductive gels, ionic gels, conductive inks, and non-metallic conductive materials.

In the present invention the term "source electron" refers to the electrode from where carriers, such as electrons or holes, flow into the gate region. As used herein the "drain electrode" refers to the electrode where the carriers flow from the gate regionconductive materials.

Therefore, in other preferred embodiment the source electron and/or the drain electrode material is selected from a list consisting of: silver, gold, copper, carbon, graphite ink, conductive fabrics, conductive textiles, metals, conductive materials, conductive polymers, conductive gels, ionic gels, conductive inks, and non-metallic conductive materials.

The "gate electrode" refers to the electrode that modulates the current in the gate region by applying a voltage to the said region, commonly known as the reference electrode. In a preferred embodiment of the biosensor of the invention, the gate electrode is composed of silver, preferably a silver wire coated in a thin layer of silver chloride, gold, platinum, or silver coated with silver oxide, or of glass-like carbon. When composed of a surface, the gate electrode is disposed as coplanar to the sensor's plane, placed in close vicinity to the graphene exposed region and in the same plane, and an area which is commonly much larger than the area of the graphene exposed region.

In other preferred embodiment of the biosensor of the invention, the gate electrode comprises a gold surface, placed at a distance less than 100 µm to the graphene exposed area, and of an area at least 100 times the area of the graphene exposed area.

The source electrode and the drain electrode, as previously mentioned, are electrically connected through the graphene sheets.

As used herein, the term "graphene" refers to an atom-thick carbon allotrope with the carbon atoms arranged in a two-dimensional honeycomb lattice. The carbon atoms are covalently bonded, forming a six-member carbon ring. Therefore, a "graphene sheet" can be seen as, but not limited to, a monolayer (ML) of covalently bonded carbon atoms. If a graphene sheet is configured as a monolayer, multiple sheets may be stacked to form multiple layers.

In other preferred embodiment of the sensor of the invention, the graphene sheet is a single graphene monolayer.

In other more preferred embodiment of the invention sensor, the graphene sheets comprises a thickness between 1 to 20 ML, preferably between 1 to 10 ML and more preferably between 1 to 4 ML.

In other more preferred embodiment of the biosensor, the graphene exposed area measures 25 µm × 75 µm. As previously mentioned, the gate electrode is placed in the close vicinity to the graphene sheets.

In other preferred embodiment, the gate electrode is placed around the source electrode, drain electrode, and graphene sheets, forming a ring sector.

Further, the graphene sheet of the G-FET biosensor of the invention can be covalently functionalized with molecules, particularly organic molecules, that can be linked to aptamers capable of detecting the specific target biomolecule.

So, in other preferred embodiment the graphene sheet of the G-FET biosensor of the invention is functionalized with an organic molecule. In other more preferred embodiment the graphene sheet of the G-FET biosensor of the invention is functionalized with an organic molecule, wherein said organic molecule comprises at least one amino group (to be incorporated to the graphene).

In other more preferred embodiment, the organic molecule further comprises at least another functional group selected of the list consisting of: thiol, carboxyl, aldehyde, epoxy and any combination thereof. In other even more preferred embodiment the organic molecule comprises at least an amino group and at least a thiol group.

In other even more preferred embodiment of the invention, the graphene sheet is functionalized with aminothiophenol (ATP).

Following the protocol of PCT/ES2017/070518, the graphene layer of the G-FET can be functionalized with aminothiophenol (ATP) molecules. The functionalization protocol is a two-step process: first, single-atom vacancies are created in the graphene layer by bombarding the surface with a noble gas ion beam and second, the G-FET with vacancies is exposed to ATP molecules (R. Bueno et al. ACS Omega, 4 (2019) 3287). In this way the ATP molecule is covalently linked to the graphene layer, ready to bind to the desired aptamer, through disulphide bridges, for the detection of the target biomolecule.

Further, the biosensor of the invention can comprise a passivation layer. So in other preferred embodiment of the invention, the biosensor further comprises a passivation layer wherein said passivation layers covers the source and drain electrodes (on top of the source and drain electrodes connected with the graphene sheet). Preferably, the passivation layer covers (or coats) the sources and drain electrodes but does not cover the graphene channel, that is the places where the graphene is not on top of (or not in contact with) the sources and drain electrodes.

The "passivation layer" as used herein refers to a non-conductive water-tight material less affected or corroded by the environment which covers the source electrode and the drain electrode to avoid electrical interactions between these electrodes and the electrolytic solution. The passivation layer is a microcoating or a light coat of a protective material to create a shell against corrosion for strengthening and preserving the source and drain electrodes. The passivation can be, non exclusively, composed of silicon oxide, silicon nitride, silicon oxynitride, amorphous carbon, epoxy resin, phenol-formaldehyde resin.

In a preferred embodiment of the sensor, the passivation layer is made of a material selected from a list consisting of: silicon oxide, silicon nitride and silicon oxynitride.

The G-FET biosensor of the invention is used to detect viral capsids, particularly the HCV core protein, that combines a biological or biomimetic component (used as a probe molecule) with a physicochemical detector (signal transducer). In the present invention, the biological component is an aptamer and the physicochemical detector is the G-FET.

The term "aptamer" as used herein refers to an *in vitro* nucleotide sequence (or analogous nucleotide sequence) capable of selectively binding to a ligand (either contained, secreted, or excreted by a target biological factor) that may be present either on the surface or in the interior of said target biological factor.

As a person skilled in the art knows, the nucleotide sequence of the aptamer in the biosensor of the invention is a single-stranded olgonucleotide (either ssDNA or ssRNA) that can include one or more chemical modifications at either the sugar motifs or its nucleobases, or even at its 5' and/or 3' ends. The aptamer can also consist of nucleic acid analogues (e.g., LNA, PNA, GNA, TNA, etc), or combinations of them with natural nucleic acids (e.g., a DNA oligonucleotide including some LNA monomers).

In a preferred embodiment of the biosensor of the invention, the aptamer comprises or consists of SEQ ID NO: 1 (5'-3'sequence of the Apt-HCVcore_01).

SEQ ID NO: 1

GCGGATCCAGACTGGTGTGCCGTATCCCTCCCTTGTAATTATTTGTTCCATCCGTTC

CGCCCTAAAGACAAGCTTC

As a person skilled in the art knows, the aptamer of the biosensor of the invention can be the complementary reverse sequence of the SEQ ID NO: 1. The terms "complementary reverse sequence" or "reverse complement sequence" used herein refers to the nucleotide sequence that is formed by assembling all of the complementary nucleotides corresponding to the aptamer of the invention, positioned in the reverse orientation with respect to the meaning sequence.

Thus, in other preferred embodiment of the biosensor of the invention, the aptamer comprises or consists of SEQ ID NO: 2 (5'-3' complementary reverse sequence of the Apt-HCVcore_01).

SEQ ID NO: 2
GAAGCTTGTCTTTAGGGCGGAACGGATGGAACAAATAATTACAAGGGAGGGATACGG
CACACCAGTCTGGATCCGC

To be able to bind the aptamer comprising SEQ ID NO: 1 to the graphene sheet of the G-FET, the graphene sheet must be functionalized.

The expression "the graphene sheet is functionalized with at least one aptamer comprising a nucleotide sequence according to SEQ ID NO:1" of the biosensor of the invention refers to the process by which the aptamer is attached to the graphene sheet. This process consists of modifying both the aptamer as well as the graphene sheet as to form a covalent bond between the two. The functionalization of graphene with an aptamer is a two-step process. In a first stage, an organic molecule is covalently incorporated in the graphene sheet. This is accomplished by bombarding the surface with a beam of noble gas ions, creating vacancies on the surface, and then, exposing that defective surface to the organic molecules. In a second step, the aptamer (upon modification of its 5'-end with a thiol group) is linked to the graphene sheet through the formation of stable disulphide bridges with the organic molecule. The organic molecules linked to the graphene are those that incorporate an amino group and a thiol group, as for example aminothiophenol (ATP).

In the present invention, the aptamer is modified with an organic molecule at its 5' end. Therefore, in a preferred embodiment of the biosensor of the invention the aptamer further comprises an organic molecule at its 5' end.

In other preferred embodiment of the biosensor of the invention, the aptamer comprises an organic molecule at its 5' end selected of the lists consisting of: an amino, a thiol group, an amino group, a carboxyl group and an epoxy group, preferably a thiol group.

Methods to add organic molecules to the 5' end of either ssDNA or RNA molecules are well known in the art and examples of such methods can be found in the literature (Ramon Eritja (Ed.), Nucleic Acids Chemistry: Modifications and Conjugates for Biomedicine and Nanotechnology. Ed. De Gruyter, 2021). Once the aptamer contains an organic molecule at its end, the graphene sheet must be modified so a covalent bond to be formed between the sheet and an organic molecule. The inventors of the present invention used a method disclosed in the European Patent number EP3486214 B1.

Therefore, in a preferred embodiment of biosensor of the invention the graphene sheet is functionalized with an organic molecule that incorporates an amino group and a thiol group, and the aptarmer is modified with a molecule that incorporates a thiol group.

The biosensor of the invention finds use in detecting HCV because the aptamer covalently linked to the functionalized graphene sheet targets the HCV core protein, which forms the virus capsid, and binds to said target with a high affinity and specificity.

Hence, another aspect of the present invention relates to the use of the biosensor of the invention for the *in vitro* detection of HCV , form here onwards the "use of the invention".

The term "hepatitis C virus" or its acronym "HCV" as used herein refer to a small (55-65 nm in size), enveloped, positive-sense single-stranded RNA virus of the family *Flaviviridae.* HCV is the cause of hepatitis C and some cancers such as liver cancer (hepatocellular carcinoma, abbreviated HCC) in humans. The HCV particle or virion consists of a lipid membrane envelope, inside which is an icosahedral capsid that is 33 to 40 nm in diameter, inside which is the RNA genome of the virus. The icosahedral capsid is formed entirely by the HCV core protein, the target of the aptamer present in the biosensor of the invention.

The term "HCV core protein" as used herein refers to the protein that forms the capsid of the virus. As with most viruses, the HCV core protein is the first part of the genome polyprotein (Uniprot access number: P27958) a large protein which results of the translation of the RNA genome of the virus. The genome polyprotein is then cleaved into the several structural and functional components required for the genomic RNA replication, virus particle formation and packaging of the virus. The HCV core protein is first cleaved into the HCV core precursor protein which corresponds to the amino acids 2 - 191 of the genome polyprotein. After this the HCV core protein matures into the final polypeptide which corresponds to the amino acids 2 - 177 of the genome polyprotein. The aptamer of the present invention recognizes both forms of the HCV core protein, particularly the region between 1 to 122 amino acids.

Another aspect of the present invention relates to a method for the *in vitro* detection of HCV, form here onwards the method of the invention, comprising:
a) providing at least one biosensor of the invention;
b) providing a electrolyte sample from a subject in contact with the biosensor of step a);
c) performing at least one measurement of an electrical signal by the biosensor of step a) in contact with the electrolyte ample of step b),
wherein a change of the electrical signal in step c) as a function of time indicates the recognition and binding between the aptamer of the biosensor and the HCV core protein present in the electrolyte sample and it is indicative of the presence of HCV.

The terms "hepatitis C virus", "HCV", "biosensor", "aptamer", "electrolyte" and "HCV core protein" have been previously defined and said definitions are equally valid for the present aspects as well as its embodiments.

The term "electrolyte sample" as used herein refers to part, fraction, portion, or subvolume considered to be representative of a larger volume of the material/object/volume to be sampled and an electrically conducting solution.

In the present invention, preferably the electrolyte sample is a isolated biological sample.

In a preferred embodiment of the method of the invention the biological sample is selected from a list consisting of: blood, plasma, serum, urine, saliva, mucus, interstitial fluid, amniotic fluid, lymphatic fluid, pericardial fluid, peritoneal fluid, tears.

The term "subject" as used herein refers to an animal, preferably a human. Therefore, in a preferred use of the biosensor of the invention, the subject is an animal, preferably a human.

The expression "providing an electrolyte sample from a subject in contact with the biosensor of step a)" of step b) of the method of the invention refers to the process by which an sample, which is a solution, is placed in the electrolyte-gated region of the biosensor of the invention as to come into contact with the functionalized graphene sheet and the gate electrode.

As used herein, the term "signal" is intended to mean an indicator that represents information. Signals include, for example, an electrical signal, and electrochemical signal and an optical signal. The term "electrical signal" refers to an indicator of an electrical quality that represents information. The indicator can be, for example, current, voltage, tunneling current, resistance, electrostatic potential, conductance, or a transverse electrical effect. An "electronic current" or "electric current" refers to a flow of electric charge. For example, an electrical signal may be an electric current passing through the graphene sheet, and the electric current may flow when an electric potential difference is applied across the graphene sheet.

In a preferred embodiment of the method of the invention, the electrical signal is selected from a group consisting of: conductance, capacitance, electrostatic potential, resistance, intensity of the current flowing into the sensor, inductance, and value of the voltage applied to the gate electrode that minimizes or maximizes any of the above quantities. When the signal used is the value of the voltage applied to the gate to minimize or maximize a quantity, the measurement is performed by changing the voltage applied to the gate in a range commonly considered between -1.2 V and 1.2 V, measuring the quantity for several voltage points applied to the gate, and determining which value of the voltage applied to the gate electrode caused the extremum value of the measured quantity.

The expression "performing at least one measurement of an electrical signal by the biosensor of step a) in contact with the electrolyte sample of step b)" of the method of the invention refers to detecting, receiving, and/or reading an electrical signal from an electrode of the biosensor of the invention when said biosensor contains an electrolyte sample in the electrolyte-gated region which contacts both the graphene sheet and the gate electrode.

The method of the invention allows the HCV detection in electrolyte samples because, upon the aptamer binding to the HCV core protein, the graphene sheet electrical properties are altered, leading to a modification of the electrical signal detected by the biosensor of the invention, thus indicating the presence of HCV in the electrolyte sample and therefore on the subject from which the sample was obtained. This modification of the electrical signal occurs in time, as aptamers (non-covalently) bind and unbind to the HCV core proteins present in the electrolyte sample. This is the process to which the expression "wherein a change of the electrical signal in step c) as a function of time indicates the union between the aptamer of the biosensor and the HCV core protein and it is indicative of the presence of HCV" of the method of the invention refers to.

The detection of HCV by the method of the invention is very sensitive and specific, as can be seen in the Examples. The method of the invention allows the detection of HCV core protein in an electrolyte sample at the attomolar to nanomolar range. Therefore, in a preferred embodiment of the method of the invention, the electrolyte sample contains HCV core protein in a concentration of at least 10⁻¹⁸ molar.

The method of the invention have clear uses in the diagnosis of infection by HCV.

Therefore, another aspect of the present invention relates to the use of the biosensor of the invention in the *in vitro* diagnosis of infection by HCV.

The term "diagnosis" as used herein refers to the procedure by which a specific disease, nosological entity, syndrome, or any health-disease condition is identified by analysing a series of clinical parameters or symptoms characteristic of said disease, and which distinguish it from other diseases with similar clinical pictures. By "diagnosis" is also intended to refer to providing information useful for diagnosis. In the present invention the health condition to diagnose is infection by HCV and the clinical parameter is the presence of HCV in a sample from a subject to be diagnosed.

### DESCRIPTION OF THE DRAWINGS

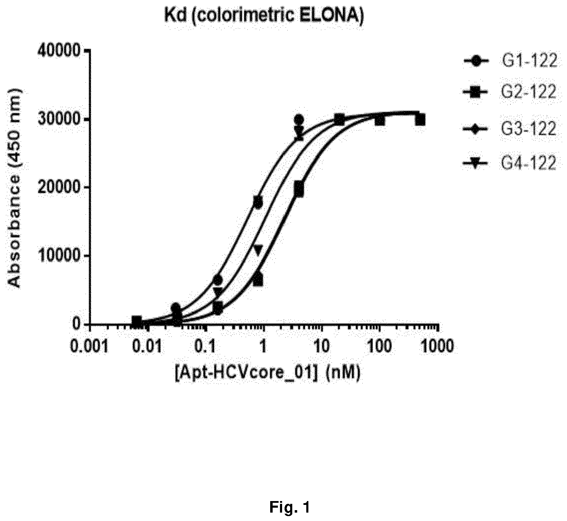

**Figure 1****.** Characterization of the affinity of aptamer SEQ ID NO: 1 (Apt-HCVcore_01) against the 122 aa-long versions of the HCV core protein belonging to viral genotypes 1 (termed G1-122), 2 (G2-122), 3 (G3-122) and 4 (G4-122) in solution. Colorimetric ELONA was used to quantify the Kd values, which resulted as follows: G1-122, Kd = 0.82 nM; G2-122, Kd = 2.4 nM; G3-122, Kd = 2.24 nM; G4-122, Kd = 1.06 nM.

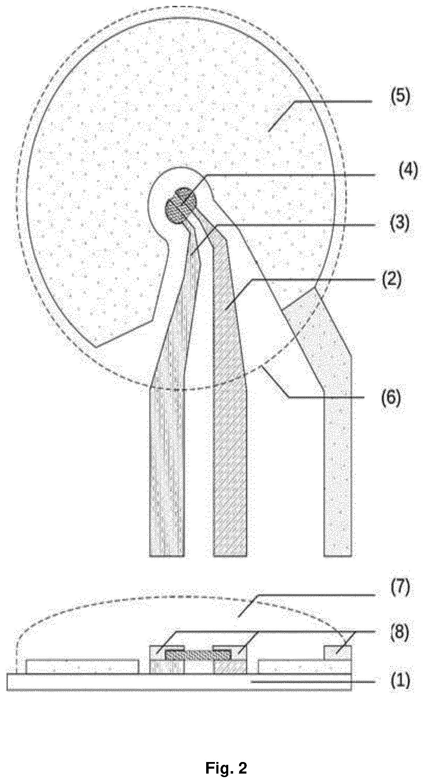

**Figure 2****.** Arrangement of the biosensor on the electrically insulating substrate (1). The biosensor of the invention is composed of the source electrode (2) and drain electrode (3) connected by the graphene sheets (4) and surrounded by the gate electrode (5) in the shape of a ring sector. The electrodes, disposed on the substrate in the electrolyte-gated region (6) where the electrolyte (7) is located, are connected to elongated wires, which bring the electrical signals to be measured by the instrumentation to the sensor edge. The source electrode, drain electrode, and connection wires of the source, drain, and gate electrodes are covered with a passivation material (8).

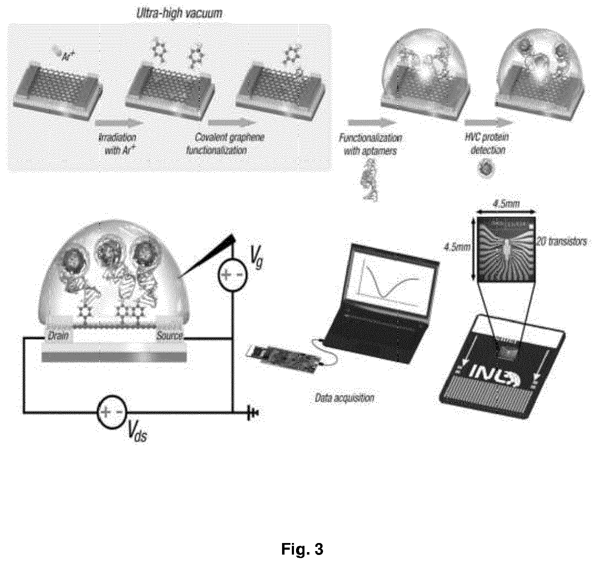

**Figure 3****.** Scheme of the stepwise fabrication and use of the biosensor of the invention. Top image shows the different stages of the device development: irradiation with noble gas ions (Ar⁺ in the image) for the creation of surface vacancies in the graphene layer, covalent graphene functionalization by exposing the surface to organic molecules, functionalization with aptamers through disulphide bridges and HCV detection. Bottom image exhibited the main elements of the G-FET based biosensor and the characterisitcs of the electrical measurements and the used transistors.

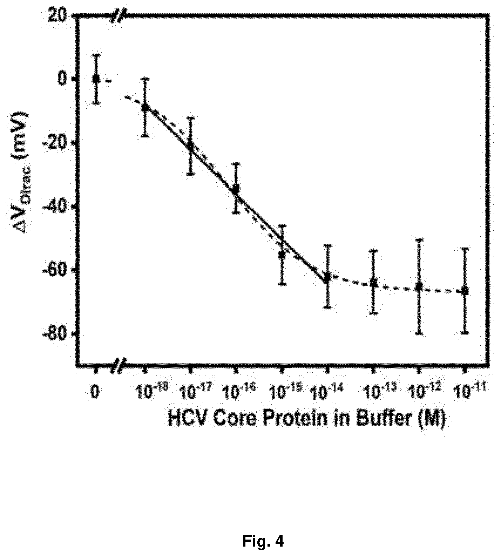

**Figure 4****.** Evolution of the signal corresponding to the Dirac voltage (ΔV_{Dirac}) of the device. The biosensor of the invention is exposed to different concentrations of the HCV core protein (between 10⁻¹⁸ and 10⁻¹¹ M). The curve shows a linear response for protein concentrations from 10⁻¹⁸ to 10⁻¹⁴ M, enabling its use as a highly sensitive and specific biosensor.

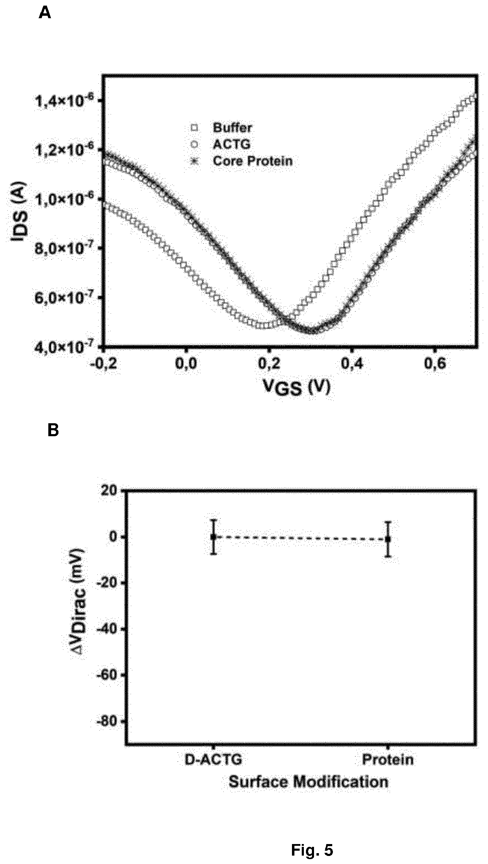

**Figure 5****.** Control experiment using the biosensor functionalized with a different, unspecific aptamer, D-ACTG. The graphene sheet functionalized with the organic molecule was exposed to a different aptamer, D-ACTG, to check aptamer specificity against HCV core protein. In addition, this system was exposed to the HCV core protein for this control experiment. a) Curves with open circles and asterisks show no differences between the response for D-ACTG sensor and D-ACTG sensor after exposition against core protein. This result evidences the affinity of the aptamer Apt-HCVcore_01 for HCV core protein, which is not the case for D-ACTG. b) The graph shows the same information encoded in gate voltage changes at the graphene Dirac point for the sensor response to the D-ACTG and after its exposure to HCV core protein.

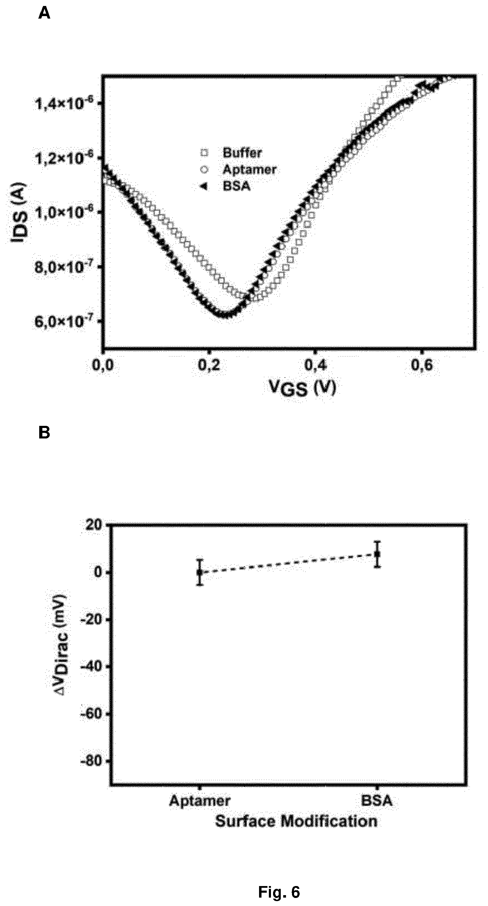

**Figure 6****.** Evaluation of the specificity of the biosensor by exposing it to an unspecific protein. The biosensor including aptamer Apt-HCVcore_01 was exposed to an unspecific protein, namely bovine serum albumin (BSA), to check the putative detection of false positives. a) The curve corresponding to the device with the aptamer (open circles) is very similar and superimposable to that of the device with the same aptamer and exposed to the BSA protein (solid triangles). b) The graph shows the same information encoded in gate voltage changes at the graphene Dirac point to respond to the aptamer Apt-HCVcore_01 and for the aptamer after exposure to the BSA protein.

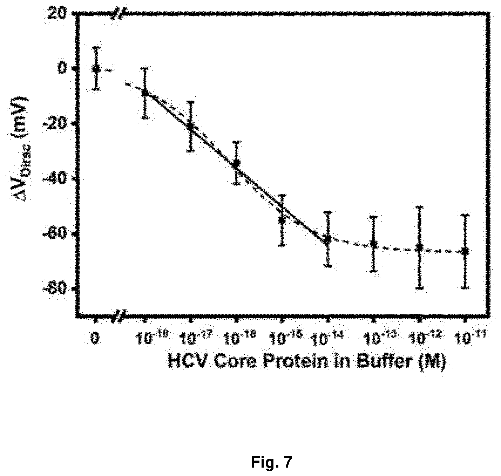

**Figure 7****.** Specific detection of HCV core protein in human plasma supplemented with such protein at different concentrations. The biosensor of the invention is exposed to human plasma containing different concentrations of HCV core protein in the range of 10⁻¹⁸ to 10⁻¹¹ M. The curve shows a linear response of the device for concentrations from 10⁻¹⁸ to 10⁻¹⁴ M, thus indicating that the biosensor with Apt-HCVcore_01 aptamer allows the specific detection of HCV core protein with high sensitivity, even in the presence of the complex mixture of proteins and other biomolecules present in human plasma.

### EXAMPLES

### Characterisation of the aptamer against HCV core

The inventors have developed specific aptamers against molecular targets of this virus, specifically against the protein of the viral capsid (core protein). Aptamers are oligomers of single-stranded nucleic acids (ssDNA or RNA), obtained using *in vitro* selection systems starting from combinatorial populations of nucleic acids, the three-dimensional structure of which enables them to bind with high affinity and specificity to the desired target molecule.

Specifically, 76 nucleotides long, ssDNA and RNA aptamers were selected *in vitro* after following a methodology analogous to that previously described (Moreno et al., 2019 Molecules, 24, 1213) and using as target molecule the HCV core protein belonging to each of the four main genotypes of the virus: HCV-1, HCV-2, HCV-3 and HCV-4. In particular, 12 and 14 rounds of *in vitro* selection (including counter-selection steps to increase the specificity of the process) were used to obtain ssDNA and RNA aptamers, respectively.

The characterisation of the affinity and specificity of the aptamers for the targets thereof was performed using different techniques, among them those based on ELONA (a system analogous to ELISA but using aptamers instead of antibodies). The functional analysis of ssDNA aptamers involved ELONA coupled to colorimetric methods. With that aim, different concentrations of HCV core protein were immobilized onto chemically modified multiwell plates, and a 5' digoxigenin-labelled version of the ssDNA aptamer was used, coupled to an anti-digoxigenin antibody labelled with horseradish peroxidase (HRP) which can trigger a colorimetric reaction. In turn, ELONA coupled to real time quantitative RT-PCR (using SYBR Green as dye) allowed analysing the performance of RNA aptamers. In both cases, the corresponding calibration curves were previously obtained, with which the affinity of each aptamer (expressed as the dissociation constant, Kd) was quantified.

One of the ssDNA aptamers that demonstrated affinity for the HCV core protein in the nanomolar range was Apt-HCVcore_01 (Kd = 0.82 nM for G1-122, Kd = 2.4 nM for G2-122, Kd = 2.24 nM for G3-122, and Kd = 1.06 nM for G4-122; Figure 1). This aptamer has the following nucleotide sequence (in the 5'-3' direction):

SEQ ID NO: 1 (Apt-HCVcore_01)
GCGGATCCAGACTGGTGTGCCGTATCCCTCCCTTGTAATTATTTGTTCCATCCGTTC
CGCCCTAAAGACAAGCTTC

In order to immobilize the aptamer on ATP-functionalised graphene for the construction of G-FET biosensor, the inventors introduced a free thiol group at its 5' end, which rendered the following chemical formula: HS-(CH₂)₆-PO₄-5'-aptamer-3'.

### Construction of G-FET biosensor

The biosensor of the present invention is based on an electrolyte-gated graphene field-effect transistor (G-FET). The G-FET devices are composed of three electrodes (gate, source, and drain) coupled to a single graphene layer between the source and the drain, and they are electrolyte-gated. Figure 2 shows the main parts of the biosensor of the present invention and its arrangement on the substrate. In the case of the present invention, the graphene layer used has been functionalised with aminothiophenol (ATP) molecules following the functionalisation protocol of the patent PCT/ES2017/070518. The functionalization protocol of G-FET is a two-step process that takes place in a UHV chamber (pressure lower than 1·10⁹ mbar). In the first step single-atom vacancies are created in the graphene layer by bombarding the surface with a noble gas ion beam at a pressure between 1·10⁻⁷ mbar and 1·10⁻¹ for a period of time between 30 s and 120 s, wherein the energy of the noble gas ions is between 100 eV and 140 eV. In the second step, the G-FET with the vacancies is subjected to an exposure to ATP molecules greater than 10 Langmuirs. The ATP molecules covalently linked to the lattice of the graphene using said protocol were exposed to the thiolated aptamer Apt-HCVcore_01 with which it forms stable disulphide bridges, the aptamer being accessible as a probe with sensor capacity. Figure 3 schematizes the stepway protocol used to fabricate and use the biosensor of the invention.

### Detection of the HCV core protein

For the analytic detection of the HCV core protein, the biosensor described above was exposed (with the folded aptamer, which comprises the sequence SEQ ID NO: 1, linked to the functionalised graphene) to contact with either a buffer solution (composed of 100 mM Hepes pH 7.4, 100 mM NaCl, 1 mM MgCl₂) or human plasma that contains HCV core protein (belonging to viral genotype 1, G1-122) at different concentrations. The G-FET biosensor converts the signal obtained when the bio identification is produced (the specific aptamer-core protein interaction on the surface) into an electrical response. When this interaction is produced, the graphene channel conductance and the general response of the device are altered, producing the biodetection.

Figure 4 shows the evolution of the signal corresponding to the Dirac voltage (ΔV_{Dirac}) of the device, consisting of the functionalized graphene linked to the aptamer Apt-HCVcore_01, exposed to different concentrations of the target HCV core protein (between 10⁻¹⁸ and 10⁻¹¹ M). From this curve, the linear response of the device to a range of concentrations covering at least 5 orders of magnitude is deduced, enabling its use thereof as a highly sensitive and specific biosensor. Furthermore, the results from the said figure show that the protein detection is already performed on an attomolar scale.

As a confirmation that there would not be any unspecific interaction between HCV core protein and a 76 nucleotides-long ssDNA sequence not selected *in vitro* against said target protein, functionalized graphene was covalently coupled to the ssDNA oligonucleotide termed D-ACTG (SEQ ID NO: 3), which contains at its 40 nucleotides-long, central region 10 repetitions of the tetranucleotide ACTG, the complete sequence thereof being (with 76 nucleotides, in the 5'-3' direction):

SEQ ID NO: 3 (D-ACTG)

GCGGATCCAGACTGGTGTACTGACTGACTGACTGACTGACTGACTGACTGACT

GGCCCTAAAGACAAGCTTC

Then, the G-FET biosensor with D-ACTG was exposed to the core protein to quantify the response thereof. This control experiment produced the expected negative response since the result was a null detection, as seen in Figure 5A. The curves obtained for D-ACTG alone (open circles) and after its exposure to HCV core protein (asterisks) gave a similar response in the value of the minimum conductance voltage. Figure 5B shows explicitly how the value of the ΔV_{Dirac}, is the same for D-ACTG whether the core protein is absent or present.

Moreover, the inventors evaluated the possibility of a false detection being given, resulting from the interaction of aptamer Apt-HCVcore_01 with an unspecific protein. For this reason, the biosensor with aptamer Apt-HCVcore_01 was exposed to the protein bovine serum albumin (BSA). This control experiment produced the expected negative result giving a null detection, as seen in Figure 6A: The curves corresponding to the biosensor with aptamer Apt-HCVcore_01 (open circles) and to the device with aptamer and exposed to the BSA protein (solid triangles) gave very similar results. Figure 6B clearly shows the close-to-zero value in the Dirac point shift for the aptamer and the aptamer exposed to the BSA protein.

Finally, to evaluate the specificity of the detection in a relevant biological fluid, the system with aptamer Apt-HCVcore_01 was exposed to human plasma, which contains a very large variety of proteins and other biomolecules, wherein the HCV core protein had been added in different concentrations (between 10⁻¹⁸ and 10⁻⁹ M).

The result of this experiment is shown in Figure 7, wherein it proves the efficient and specific HCV core protein detection with a result similar to the one obtained with the target protein in buffer solution (Figure 4) Therefore, the biosensor of the invention (schematized in Figures 2 and 3) allows the detection of HCV core protein in human plasma with high efficiency (attomolar level) and specificity (no side effects produced by any of the proteins or other biomolecules contained in the plasma).

## Claims

1. An electrolyte-gated graphene field-effect transistor (G-FET)-based biosensor **characterized in that** said biosensor is functionalized with at least one aptamer comprising a nucleotide sequence according to SEQ ID NO:1.

2. The G-FET biosensor according to claim 1 wherein the biosensor comprises:
a) an electrically insulating substrate (1);
b) a gate electrode (5) on the top of the electrically insulating substrate (1), wherein said gate electrode (5) contains at least one electrolyte-gated region (6) wherein an electrolyte (7) is arranged thereof;
c) a source electrode (2) and a drain electrode (3) on top of the electrically insulating substrate (1) and in the electrolyte-gated region (6), without direct contact between them (2, 3);
d) at least one graphene sheet (4) located between the source electrode (2) and the drain electrode (3) in the electrolyte-gated region (6) wherein said graphene sheet (4) is configured to electrically connect the source electrode (2) to the drain electrode (3);
wherein the electrolyte (7) is exposed to the graphene sheet (4) and the gate electrode (5); **characterized in that** the graphene sheet is functionalized with at least one aptamer comprising or consisting of a sequence according to SEQ ID NO:1.

3. The biosensor according to claim 1 or 2 wherein the electrically insulating substrate is silicon coated with silicon oxide or silicon nitride.

4. The biosensor according to any one of claims 1 or 3 wherein the source and/or drain electrodes are made of a material selected from a list consisting of: silver, gold, copper, carbon, graphite ink, conductive fabrics, conductive textiles, metals, conductive materials, conductive polymers, conductive gels, ionic gels, conductive inks and non-metallic conductive materials.

5. The biosensor according to any one of claims 1 or 4 wherein the gate electrode is composed of silver, preferably a silver wire coated in a thin layer of silver chloride, gold, platinum, or silver coated with silver oxide or of glass-like carbon.

6. The biosensor according to any one of claims 1 to 5 wherein the graphene sheet is a graphene monolayer (ML), preferably the graphene monolayer comprises a thickness between 1 to 20 ML, preferably 1 to 4 ML.

7. The biosensor according to any one of claims 1 to 6 wherein the graphene sheet is functionalized with an organic molecule, preferably wherein the organic molecule contains an amino group and a thiol group.

8. The biosensor according to claim 7 wherein the organic molecule is aminothiophenol (ATP).

9. The biosensor according to any one of claims 1 to 8 further comprises a passivation layer (8) wherein said passivation layer covers the source electrode (2) and drain electrode (3) connected with the graphene sheet (4).

10. The biosensor according to claim 9 wherein the passivation layer (8) is made of a material selected from a list consisting of: silicon oxide, silicon nitride and silicon oxynitride.

11. The biosensor according to any one of claims 1 to 10 wherein the aptamer further comprises a thiol group at its 5' end.

12. Use of the biosensor according to any one of claims 1 to 11 for the *in vitro* detection of the hepatitis C virus (HCV).

13. A method for the *in vitro* detection of hepatitis C virus comprising:
a) providing at least one biosensor according to claims 1 to 11;
b) providing an electrolyte sample from a subject in contact with the biosensor of step a);
c) performing at least one measurement of an electrical signal by the biosensor of step a) in contact with the electrolyte sample of step b),
wherein a change of the electrical signal in step c) as a function of time indicates the union between the aptamer of the biosensor and the HCV core protein present in the electrolyte sample and it is indicative of the presence of HCV.

14. The method according to claim 13 wherein the electrolyte sample is an isolated biological sample, preferably wherein the biological sample is selected from the list consisting of: blood, plasma, serum, urine, saliva, mucus, interstitial fluid, amniotic fluid, lymphatic fluid, pericardial fluid, peritoneal fluid and tears.

15. The method according to claims 13 or 14 wherein the subject is an animal, preferably a human.
